(19) Europäisches Patentamt
European Patent Office
Office européen des brevets

(11) Publication number: **0 128 458 A2**

# (12) EUROPEAN PATENT APPLICATION

(21) Application number: 84106148.4

(22) Date of filing: 16.05.84

(51) Int. Cl.³: C 07 C 37/62, C 07 C 39/36, C 07 C 39/27, A 01 N 37/18, C 07 C 103/34

(30) Priority: 17.05.83 US 495382

(43) Date of publication of application: 19.12.84 Bulletin 84/51

(84) Designated Contracting States: AT BE DE FR GB IT NL

(71) Applicant: THE DOW CHEMICAL COMPANY, Dow Center 2030 Abbott Road Post Office Box 1967, Midland Michigan 48640 (US)

(72) Inventor: Louks, David H., 7597 Laurie Lane South, Saginaw Michigan 48603 (US)
Inventor: Thompson, Leonard R., 2261 E. Ashby, Route 7, Midland Michigan 48640 (US)
Inventor: Muench, Wayne C., 5312 Gardenbrook, Midland Michigan 48640 (US)

(74) Representative: Weickmann, Heinrich, Dipl.-Ing. et al, Patentanwälte Dipl.-Ing. H.Weickmann Dipl.-Phys.Dr. K.Fincke Dipl.-Ing. F.A.Weickmann Dipl.-Chem. B. Huber Dr.-Ing. H. Liska Dipl.-Phys.Dr. J. Prechtel Möhlstrasse 22, D-8000 München 80 (DE)

## (54) Preparation of halogenated phenols, novel complexes of phenols and use in controlling microbial growth.

(57) Halogenated phenolic compounds are prepared by forming a low melting point complex of the phenolic compound and contacting the low melting point complex with a halogenating agent under halogenating conditions.

EP 0 128 458 A2

# PREPARATION OF HALOGENATED PHENOLS, NOVEL COMPLEXES OF PHENOLS AND USE IN CONTROLLING MICROBIAL GROWTH

The present invention relates to the preparation of halogenated phenolic compounds, novel complexes of phenols and use in controlling microbial growth. More particularly the present invention relates to an improved process for the direct halogenation of phenolic compounds whereby reduced amounts of by-products, particularly halogenated dioxin compounds are also prepared.

Halogenated phenols such as chlorinated phenols, especially tetrachlorophenols and pentachlorophenol, are valuable fungicides that are conveniently prepared by the direct halogenation of phenol or halogenated phenols in the presence of various catalysts such as $AlCl_3$, $FeCl_3$, activated carbon and quinoline, tellurium and various salts thereof, as well as such catalysts in combination with various promoters such as those taught in USP 4,376,220. The preferred catalyst is aluminum trichloride.

In the case of chlorinated phenols, the reaction is conducted neat at a starting temperature of 65°C--130°C. After three or four chlorine atoms have been substituted onto the phenol, the temperature of the reaction mixture is increased to maintain a temperature approximately 10°C above the melting point of the chlorinated mixture. The reaction is normally complete in 5-15 hours. Because the mixture's melting point increases during the later stages of the chlorination, the preparation of pentachlorophenol may require reaction temperatures of up to 190°C or even higher so that the reaction mixture remains in a liquid state. It is now known that elevated temperatures necessary for the preparation of highly chlorinated phenols are particularly conducive to the formation of increased amounts of undesirable by-products particularly chlorinated dibenzo-p-dioxins.

In USP 2,131,259, it is further taught to employ solvents such as chlorinated aliphatic compounds in the reaction, thereby allowing the use of lower reaction temperatures while maintaining a liquid reaction mixture. Suitable solvents included ethylene dichloride, sym-tetrachloroethane, trichloroethane, carbon tetrachloride and propylene chloride. The use of such solvents has not proven to be practical in commercial usage due to the possible formation of solvent-reactant by-products as well as the increased production costs associated with removal and recycle of solvent during the process, and decreased effectiveness of the chlorination process due to the solvent's dilution effect.

According to the present invention there is provided an improved process for halogenating phenolic compounds comprising:

(1) forming a low melting point complex of the phenolic compound; and

(2) contacting the low melting point complex of the phenolic compound with a halogenating agent under halogenation conditions.

The phenolic compounds suitably halogenated by the present process include any aromatic compound containing phenol functionality that is capable of halogenation and which suitably forms a complex with the complex forming agents employed herein. Preferred are those phenolic compounds meeting the above criteria that are directly halogenated neat only at elevated temperatures. Most preferred are phenol itself as well as partially halogenated derivatives thereof. Especially preferred are phenol and all mono-, di-, tri- or tetrachlorinated phenols or mixtures thereof.

The halogenating agents suitably employed in the present invention include a halogen compound or a source thereof corresponding to the desired halogen functionality of the resulting product. A preferred halogenating agent is chlorine.

The conditions appropriately selected for the halogenation reaction are those previously known in the art. Generally, a catalytic amount of a Lewis

acid catalyst such as aluminum trichloride or ferric chloride is employed to aid in the reaction. The catalyst is employed in an anhydrous state. Elevated temperatures of from 50°C to 150°C are employed. Preferred temperatures are those at which formation of appreciable amounts of undesirable halogenated dibenzo-p-dioxins is avoided. Preferred temperatures are from 80°C to 130°C. Most preferred temperatures are from 90°C to 115°C. At such temperatures, the formation of halogenated dibenzo-p-dioxin compounds is preferably less than 50 ppm by weight and most preferably less than 10 ppm based on the weight of the halogenated phenol product. The temperature selected will be determined by the melting point of the reaction mixture keeping in mind that the mixture is desirably maintained in the molten state. Elevated or reduced pressures may be employed as is desired. Preferred pressures are atmospheric or higher.

The formation of low melting point complexes with the phenolic reactants of the present process is accomplished using any suitable complexing agent. Included are all compounds that are capable of forming stable, isolatable complexes having a melting point less than 150°C, preferably less than 130°C and most preferably less than 115°C. Identification of suitable complexing agents may be readily determined by the skilled artisan by the simple expedient of combining the phenolic compound and the complexing agent optionally in the presence of a solvent and noting the formation of a stable complex upon isolation thereof. By the term stable is meant that the complex is sufficiently durable so as to exist independently of solvents.

Suitable dialkylamides for use according to the present invention are those amides corresponding to the formula

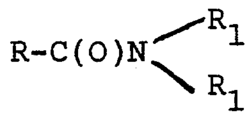

wherein R is hydrogen or $C_{1-6}$ alkyl or phenyl; and $R_1$ and $R_2$ are selected from $C_{1-6}$ alkyl groups.

Preferred complexing agents according to the present invention are the lower alkyl amide derivatives of lower aliphatic aldehydes, e.g., dimethylformamide, diethylformamide, dimethylacetamide, etc. A particularly preferred complexing agent is dimethylformamide (DMF). The melting point of the 1:1 complex of DMF and pentachlorophenol has been found to be 62°C, allowing the neat, direct chlorination of a complex of phenol and DMF to be readily accomplished at temperatures well below 150°C, or even below 130°C or 115°C.

The complex preferably comprises about a stoichiometric amount of the complexing agent chemically bound to the phenolic compound so as to form a stable isolatable species. Advantageously, from 0.5 to 1.1 equivalents of the complexing agent are employed per equivalent of the phenolic compound, preferably 0.9 to 1.5 moles of complexing agent are employed for each mole of phenolic compound and most preferably from 1 to 1.05 moles of complexing agent are employed for each mole of phenol.

After halogenation of the stable complex of the phenolic compound is completed, the product is easily recovered by simply cooling the mixture. The complex may also be recovered by dissolving in organic solvents or by any other suitable method. It has been found that the 1:1 complex of DMF and pentachlorophenol is generally more soluble in commonly employed organic solvents such as methylene chloride than is pentachlorophenol itself.

As previously explained, the complex may be prepared by first forming a complex of the dialkylamide and a lesser halogenated phenol. This complex may then be chlorinated or brominated by standard techniques such as contacting with gaseous chlorine in the liquid phase to prepare the corresponding complex of fully halogenated phenol. In this regard, it should be noted that care should be exercised in the procedure to avoid exceeding explosive limits of the chlorine/dialkylamide mixture.

The resulting complex has been found to be more soluble in solvents normally employed for solvating halogenated phenols, especially chlorinated phenols than are the neat halogenated phenols themselves. Suitable solvents for use with the present complex include chlorinated hydrocarbons, such as dichloromethane, dichloroethane, etc.; alkanes such as hexane, heptane, etc.; and other commonly employed organic solvents such as fuel oil, motor oil, etc. Due to the increased solubility of the pentahalophenol-dialkylamide complex over that of the pentahalophenol alone; solutions of the composition are capable of imparting increased antimicrobial action to cellulosic materials due to the higher loadings obtainable thereby.

As an additional advantage of the present composition it has been surprisingly discovered that on a weight basis, the present complexes, especially a complex of pentachlorophenol, impart at least equivalent antimicrobial action compared to pentachlorophenol alone in spite of replacement of part of the active pentachlorophenol component with the dialkylamide. Accordingly, especially when employing dimethylformamide, antimicrobial compositions may now be prepared having equivalent or even improved efficacy measured on a weight basis compared to pentachlorophenol, comprising in part a less expensive dialkylformamide complexed with pentachlorophenol.

The compositions are prepared and employed, e.g., to treat wood materials according to standard techniques involving no substantive departure from known and previously published methods. Suitably, the complex is added to a solvent, such as those previously described and the resulting solution contacted with the cellulosic material by dipping, painting, pressure treatment, etc. Effective amounts of the present complex incorporated into the cellulosic material range from 0.01 percent to 50 percent by weight of the cellulosic material before treatment. Preferred are amounts from 0.1 percent to 10 percent. In addition, the pentahalophenol may be recovered from the complex in pure form if desired by acidification of aqueous caustic solutions of the dialkylamide complex.

The complex may be employed in applications where previously the halogenated phenol alone has been employed. For example, it has been found that equivalent

or even improved fungicidal activity on a weight basis may be obtained by use of a complex of pentachlorophenol and DMF as compared to the use of pentachlorophenol alone.

Alternatively, the complex can be easily broken and the complexing agent and halogenated phenol recovered according to well-known chemical techniques. For example, the 1:1 complex of DMF and pentachlorophenol is readily separated by dissolving the same in aqueous base and then neutralizing the base by addition of acid. Alternatively, satisfactory recovery rates may also be obtained by simply contacting the resulting complex with water.

Use of complexes of phenolic compounds according to the present invention is to be contrasted with the use of a solvent according to prior art processes in that no stable isolatable species formed by interaction of the phenol and a solvent results when a solvent is employed.

The following examples further illustrate the invention.

Example 1

To a 3-necked, round-bottomed flask with thermowell equipped with a mechanical stirrer, a water condenser having an HCl gas scrubber attached thereto, and a chlorine addition dip tube is added 150.0 g (0.920 mole) of a mixture of 99 percent 2,4-dichlorophenol and 1 percent 2,6-dichlorophenol. After melting the dichlorophenol at 45°C using infrared heat lamps, 0.75 g $AlCl_3$

(anhydrous) and 67.3 g (0.921 mole) DMF are added and mixed. After heating with agitation to 78°C, the chlorine gas addition is started. The reaction exotherm plus additional heating raises the temperature to 100°C within 10 minutes, where the temperature is controlled for the remainder of the reaction. Chlorine is added at a rate of approximately 45 g/hr for 6.2 hours. Upon termination of the chlorine addition (after cooling to room temperature), there is recovered 365.4 g of yellow solid. The strong odor of HCl gas vented after storage of the product sample bottle suggests that considerable HCl and/or $Cl_2$ gas is absorbed in the reaction product.

The product is analyzed by use of gas phase chromatography employing an internal standard. The phenolic compounds are derivatized with bis(trimethylsilyl)-acetamide before injection. Separation is accomplished by a 20-foot, 10 percent UCW 98 column. Determination of dimethylformamide is obtained by a 10-foot column of bonded DEGS on 80/100 Chromasorb W. AW. using an external standard.

Analysis indicated that the crude product contains about 66 weight percent pentachlorophenol, 98 percent of theoretical yield. By-product content is as follows:

| | |
|---|---|
| hexachlorodibenzo-p-dioxin | 0.1 ppm |
| octachlorodibenzo-p-dioxin | 6.0 ppm |
| 2,3,7,8-tetrachlorodibenzo-p-dioxin | $<$0.1 ppm |

It is seen that very low levels of by-product halogenated dibenzo-p-dioxins are prepared according to the presently invented process.

Example 2 - Pentachlorophenol N,N-Dimethylformamide Complex

To a suspension of 26.6 g (0.1 mole) of pentachlorophenol in 25 ml of methylene chloride is added 7.7 g (0.105 mole) of N,N-dimethylformamide (DMF). Gentle swirling of this mixture effects complete solution. The solution is filtered under gentle suction to remove debris. Solvent is removed from the filtrate *in vacuo* at 25°C-35°C (to ca 15 mm) with the aid of a rotary evaporator.

The white solid that remains is recrystallized from 100 ml of cyclohexane. There is recovered 31.5 g (92.8 percent) of white needles, m.p. 61.5°C--62.5°C. The material is identified as a 1:1 complex of DMF and pentachlorophenol by nuclear magnetic resonance spectroscopy and by elemental analysis.

Example 3

The pentachlorophenol-N,N-dimethylformamide complex prepared in Example 1 is subjected to antimicrobial screening to test inhibition of growth of various test organisms. Accordingly, agar cultures are prepared containing various concentrations of inhibitor. The cultures are inoculated with various organisms further identified in Table I. The weight concentration of inhibitor

required to reduce growth of the test organism by 50 percent compared to a control culture (minimum inhibiting concentration) is recorded in Table I.

TABLE I

| Test Organism | Minimum Inhibitory conc, ppm PCP* | PCP/DMF** |
|---|---|---|
| Anaerobes | | |
| Actinomyces Viscosus | 10 | 10 |
| Clostridium Pefringens | 100 | 100 |
| Clostridium Septium | 100 | 10 |
| Bacteroides Fragilis | 100 | 100 |
| Bacteroides Multiacidus | 100 | 100 |
| Streptococcus Faecalis | 100 | 100 |
| Selenomonas Ruminantium | >500 | 500 |
| Streptococcus Mutans | 100 | 100 |
| Lactobacillus Fermentum | 150 | 100 |
| Streptococcus Bovis | >500 | 100 |
| Batyrivibrio Fibrisoluens | >500 | 100 |
| Ruminococcus Albus | >500 | 100 |
| Aerobes | | |
| Staphylococcus Aureus | 1.0 | 10 |
| Escherichia Coli | 500 | 500 |
| Salmonella Typhimurium | 100 | 500 |
| Pseudomonas Aeruginosa | >500 | 500 |
| Proteus Mirabilis | >500 | >500 |
| Candida Albicans | 100 | 100 |
| Aspergillus Niger | >500 | 100 |

*PCP - pentachlorophenol
**PCP/DMF - pentachlorophenol/N,N-dimethylformamide

It is seen by comparison of the test data that, except in two instances, the organisms are inhibited towards growth at weight concentrations of the pentachlorophenol-N,N-dimethylformamide complex equal to

or less than those of pentachlorophenol alone. Accordingly, it is seen that essentially equivalent antimicrobial effect compared to the effectiveness of pentachlorophenol is obtained by use of the present invented complex containing on a weight basis reduced amounts of pentachlorophenol.

WHAT IS CLAIMED IS:

1. A process for halogenating phenolic compounds comprising:

(1) forming a low melting point complex of the phenolic compound; and

(2) contacting the low melting point complex of the phenolic compound with a halogenating agent under halogenation conditions.

2. A process according to Claim 1 wherein the phenolic compound is phenol, a partially halogenated phenol, or a mixture thereof.

3. The process of Claim 1 wherein the halogenating agent is chlorine.

4. The process of Claim 1 wherein the halogenation is carried out in the presence of a catalyst.

5. The process of Claim 1 wherein the reaction temperature is from 50°C to 150°C.

6. The process of Claim 1 wherein the complex comprises the reaction product of the phenolic compound with about a stoichiometric amount of a dilower alkylamide derivative of a lower alkyl aldehyde.

7. The process of Claim 6 wherein the complex comprises the reaction product of the phenolic compound with dimethylformamide, diethylformamide or dimethylacetamide.

8. A composition of matter substantially free of solvent comprising a complex of a pentahalophenol and a dialkylamide corresponding to the formula:

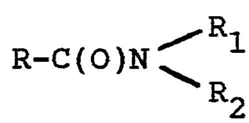

wherein R is hydrogen, $C_{1-6}$ alkyl or phenyl; and $R_1$ and $R_2$ are selected from $C_{1-6}$ alkyl groups.

9. A composition of matter according to Claim 8 wherein the pentahalophenol is pentachlorophenol and the dialkylamide is dimethylformamide.

10. A process for treating cellulosic materials to prevent microbial growth thereon comprising incorporating therein an amount effective to prevent microbial growth of a complex of any one of Claim 8 or 9.